# EUROPÄISCHE PATENTSCHRIFT

(11) **EP 1 923 038 B1**
(45) Veröffentlichungstag und Bekanntmachung des Hinweises auf die Patenterteilung: **18.05.2011**
(21) Anmeldenummer: 06023846.6
(22) Anmeldetag: 16.11.2006
(51) Int. Cl.: A61K 6/10

(54) **Silikonabformmasse mit zweistufigem Aushärtungsmechanismus**
Silicon impression material with a two-step curing mechanism
Matériau à empreinte silicone durcissable à deux étapes

(43) Veröffentlichungstag der Anmeldung: 21.05.2008
(73) Patentinhaber: Ernst Mühlbauer GmbH & Co.KG, 25870 Norderfriedrichskoog (DE)
(72) Erfinder: Böttcher, Henrik, 22335 Hamburg (DE); Neffgen, Stephan, Dr., 22459 Hamburg (DE)
(74) Vertreter: Glawe, Delfs, Moll

(56) Entgegenhaltungen:
- EP-A- 1 502 572
- WO-A-02/058641

## Beschreibung

Die Erfindung betrifft eine mischergängige Mehrkomponentenabformmasse mit zweistufigem Aushärtungsmechanismus sowie deren Herstellung.

Mischergängige Mehrkomponentenabformmassen mit zweistufigem Aushärtungsmechanismus, wie sie unter anderem in der Zahnmedizin verwendet werden, sind dem Fachmann beispielsweise aus WO 02/058641 und EP-A-1 502 572 bekannt.

Die zweistufig härtenden mischergängigen Materialien ermöglichen knetbare Materialien aus automatischen Misch- und Dosiersystemen auszutragen.

WO 02/058641 offenbart ein Mehrkomponentensystem zur Abdrucknahme, das (a) mindestens eine Verbindung mit mindestens zwei Alkenylgruppen, (b) mindestens ein Organohydrogenpolysiloxan (c) mindestens einen Hydrosilylierungskatalysator, (d₁) mindestens eine polymere Verbindung mit mindestens einer Alkinylgruppe und/oder (d₂) mindestens eine Verbindung mit mindestens einer Si-OR-Struktureinheit, wobei R= H, Alkyl, Alkoxyalkyl oder Acyl ist, und bei Anwesenheit von (d₂) (e) mindestens ein Kondensationskatalysator und/oder -vernetzer enthält. Die Verbindungen (a), (b), (d₁), (d₂) wird in einer Komponente A und die Verbindungen (c) und (e) in einer Komponente B bereitgestellt, die miteinander beispielsweise mittels eines Spendegerätes (z.B. MixStar® oder Pentamix®) gemischt werden. Dabei geht die Mischung von einer dünneren mischergängigen Ausgangskonsistenz zu einer zäheren, plastischen Phase über, in der das Material z.B. zur Zahnabformung verarbeitet wird, bevor es in einer zweiten Stufe zu seiner endgültigen elastischen Form aushärtet. Zu Beginn des Mischens weist die Mischung eine mischergängige Konsistenz von > 30 mm nach ISO 4823 auf, wonach die Mischung durch Kondensationsreaktionen von Si-OR Gruppen und/oder durch Hydrosilierungsreaktionen von Alkinylgruppen mit SiH-Gruppen in einen zweiten Zustand mit einer Konsistenz von < 30 mm nach ISO 4823 übergeht. In diesem Zustand, der üblicherweise als heavy bodied oder putty Konsistenz bezeichnet wird, bleibt die Konsistenz einen gewissen Zeitraum nahezu unverändert. In dieser Zeit wird die Mischung verarbeitet, d.h. der Abdruck abgeformt. Danach geht die Mischung durch eine Hydrosilierungsreaktion zwischen Alkenylgruppen mit SiH-Gruppen in einen dritten festen, elastischen Zustand über, in dem das Abformergebnis festgehalten wird.

EP-A-1 502 572 offenbart ein ähnliches zweistufig aushärtendes Mehrkomponentensystem, bei dem in einem ersten Schritt eine Kondensationsreaktion stattfindet der in einem zweiten Schritt die Additionsreaktion folgt. In dem Kondensationsschritt werden Siloxane mit Carbinol-, Carboxyl- und Amin-Gruppen verwendet.

Der Erfindung liegt die Aufgabe zugrunde, eine Abformmasse der eingangs genannten Art zu schaffen, die stabil ist, insbesondere langzeitstabil ist.

Die Erfindung löst diese Aufgabe durch eine Abformmasse mit den Merkmalen des Anspruch 1. Vorteilhafte Ausführungsformen der Erfindung sind in den Unteransprüchen offenbart.

Die Erfindung hat erkannt, dass die zur Realisierung in der WO 02/058641 und EP-A-1 502 572 beschriebenen zweistufigen Aushärtungsmechanismen verwendeten Zusammensetzungen Komponenten enthalten, die reaktive Gruppen tragen, die einen wesentlichen Nachteil für die Stabilität der Abformmassen bergen. In der Abformmasse der WO 02/058641 sind dies Silanol-Gruppen, in der Abformmasse der EP-A-1 502 572 sind es Hydroxyalkylgruppen, Aminogruppen oder Carboxylgruppen. Die genannten Komponenten können mit den Hydrogensiloxanen, die als Vernetzerkomponenten in den Abformmassen immer zugegen sind, unter Abspaltung von Wasserstoff reagieren. So reagieren beispielsweise die Aminogruppen und Carboxylgruppen in Gegenwart von Wasser, wie es beispielsweise über die Füllstoffkomponente als Feuchtigkeitsspur eingebracht wird, in vorgenannter Weise. Ein weiterer von vorliegender Erfindung erkannter Nachteil der Aminogruppen und Carboxylgruppen besteht darin, dass sie mit dem für Additionsreaktionen vorzugsweise verwendeten Platinkatalysator wechselwirken und eine Retardierung, d.h. zeitliche Verzögerung, bzw. eine vollständige Inhibierung der Additionsvernetzung bewirken können. Dadurch wird die Stabilität, insbesondere die Langzeitstabilität der Abformmassen beeinträchtigt.

Die Erfindung hat erkannt, dass mit einer Verbindung gemäß Merkmal (b) von Anspruch 1, die keine reaktiven Gruppen wie Hydroxyalkylgruppen, sekundäre Aminogruppen oder Carboxylgruppen aufweist, die mit einem Organohydrogenpolysiloxan oder Hydrosilylierungskatalysator wie Platin reagieren können, vorgenannte unerwünschte Reaktionen vermieden und eine Stabilität der Abformmassen über einen langen Zeitraum sichergestellt werden kann.

Gemäß einer bevorzugten Ausführungsform der Erfindung ist die chelatisierende Gruppe der Verbindung der Komponente (b) eine Dicarbonylgruppe, insbesondere eine 1,3-Dicarbonylgruppe wie ein β-Dicarbonsäureester oder β- Ketoester. Bevorzugt werden Verbindungen der Formeln

R⁵ₐR¹_{b}SiO-(SiR¹₂O)_{c}-(SiR¹R⁵O)_{d}-SiR¹_{b}-R⁶ₐ

und

R⁵ₐR¹_{b}SiO-[(SiR¹₂O)_{c}-(SiR¹R⁵O)_{d}-(R⁷)_{g}]ₕ-OSiR¹_{b}R⁵ₐ

mit
R¹= lineares oder verzweigtes Alkyl, Fluoralkyl, Cycloalkyl oder Aryl;
R²= lineares oder verzweigtes Alkylen, Fluoralkylen, Cycloalkylen oder Arylen;
R³= lineares oder verzweigtes Alkylen mit 1 bis 10 C-Atomen, Cycloalkylen oder Arylen;
R⁴= lineares oder verzweigtes Alkyl, Cycloalkyl, Aryl, NR¹₂, NHR¹ oder Alkoxy;
R⁵= R⁴-CO-R³_{f}-CO-Xₑ-R²-;
R⁶= R⁴-CO-R³_{f}-CO-Xₑ-R²;
R⁷= SiR¹₂-R²-Xₑ-CO-R³_{f}-CO-Xₑ-R²-SiR¹₂;
X= O oder NR¹;
a= 0 bis 3; b= 3-a; c= 0 bis 10.000; d= 0 bis 500; e= 0 oder 1; f= 0 oder 1; g= 1 bis 100 und h= 1 bis 1000 verwendet.

Vorzugsweise besteht die Abformmasse aus mindestens einer Komponente B (Basiskomponente) und einer Komponente K (Katalysatorkomponente), wobei die Komponente B die Komponenten (a), (b) und (c) und die Komponente K die Komponenten (d) und (e) enthält. Die Komponenten B und K sind beispielsweise Pasten, die im Mischungsverhältnis von 10:1 bis 1:1, besonders bevorzugt 5:1 angemischt werden.

Bevorzugte Alkenylverbindungen (a) der Komponente B sind solche mit der Struktur 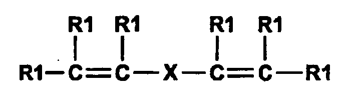 oder 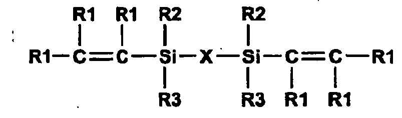 oder 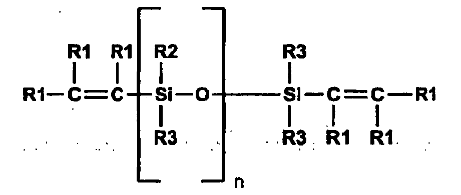 mit
n=0-20.000,
R1 = H-, Alkyl-, Aryl-, Arylalkyl-, halogensubstituierte Alkyl- und Arylgruppen, Cyanalkyl-, Cycloalkyl-, Cycloalkenyl-, sowie Kombinationen davon;
R2 = Alkenyl-, Alkinyl-, Halogen-, Aryl-, Alkylaryl-, H-, Halogensubstituierte Alkyl- und Arylgruppen insbesondere Alkyl-, sowie Kombinationen davon,
R3 = R2, oder R3 verschieden von R2, wobei R3 insbesondere Alkyl-, Methyl-, Alkinyl-, Ethinyl- oder Kombinationen davon
und
X = Polysiloxan, Oligokieselsäureester, Polykieselsäureester, Polyether, polymere Kohlenwasserstoffe, Polyester und Copolymere der oben genannten Verbindungen.

Die bevorzugte Alkenylverbindung (a) kann auch ein Silan-Dendrimer mit terminalen Alkenylgruppen sein oder als QM-Harz mit mindestens einer Si-Alkenylgruppe vorliegen.

Bevorzugte Organohydrogenpolysiloxane (c) die in der Komponente B enthalten sind, sind Polyalkyl-, Polyaryl- und Polyalkylaryl-, Polyhalogenalkyl-, Polyhalogenaryl- oder Polyhalogenalkylaryl-Siloxane. Sie können als Oligomere oder Polymere in linearer, verzweigter oder zyklischer Form oder als QM-Harze vorliegen und weisen mindestens eine Si-H-Bindung auf. QM-Harze bestehen aus Q-Einheiten (SiO_{4/2}) und M-Einheiten (Me3Si_{01/2}).

Bevorzugte Hydrosilylierungskatalysatoren (d) die in der Komponente K enthalten sind, sind die Übergangsmetalle der 8. Nebengruppe, insbesondere Platin, Palladium und Rhodium oder deren Salze, Komplexe und Kolloide, vorzugsweise Platinkomplexe und Salze der Hexachloroplatinsäure, insbesondere Platin (0)-1,3-divinyl-1,1,3,3 tetramethyldisiloxan-Komplex.

Bevorzugte Verbindungen mit einem chelatisierbaren Metallatom (e), die als Kondensationskatalysatoren bzw. Ligandenaustauschkatalysatoren fungieren und in der Komponente K enthalten sind, sind Alkoxymetallkomplexe- R₄Me, vorzugsweise R₄Ti oder R₄Zr sowie deren Oligo- oder Polykondensate.

Bevorzugt sind die Komponenten (a) - (e) in folgenden Mengenanteilen in der Abformmasse enthalten:
10 - 60 Gew.-% Komponente (a),
1 - 25 Gew.-% Komponente (b),
0,2 - 10 Gew.-% Komponente (c),
0,005 - 5 Gew.-% Komponente (d) und
0,1 - 7 Gew.-% Komponente (e).

Die Komponenten B und K können zusätzlich weitere Bestandteile, beispielsweise Inhibitoren der Additionsreaktion, die die Additionsreaktion verlangsamen oder für eine bestimmte Zeit unterdrücken, Wasser abgebende Mittel, Trockenmittel, inerte Trägerstoffe, verstärkende und nicht verstärkende Füllstoffe sowie weitere Hilfsstoffe enthalten.

Inhibitoren der Additionsreaktion sind beispielsweise offenbart in EP-A-1502572.

Als Wasser abgebende Mittel werden vorzugsweise anorganische Füllstoffe, die eine oberflächlich gebundene Restfeuchtigkeit oder im Kristallgitter gebundenes Wasser enthalten, Zeolithe oder gezielt befeuchtete Füllstoffe oder organische Stoffe mit definierten Wassergehalt eingesetzt.

Als Trockenmittel werden vorzugsweise getrocknetes Kalziumsulfat, Zeolithe, getrocknete Füllstoffe oder Wasser aufnehmende organische Verbindungen wie Oxazolidine und Alkalisalze der Poly(meth)acrylsäure eingesetzt.

Als inerte Trägerstoffe werden bevorzugt Mineralöle, verzweigte Kohlenwasserstoffe, Vaseline, Ester, Phtalsäureester, Acetyltributylcitrat, Polyalkylenoxide und Polyester und deren Copolymere verwendet.

Als verstärkende Füllstoffe werden vorzugsweise hochdisperse, aktive Füllstoffe wie Titanoxid, Aluminiumoxid, Zinkoxid, pyrogene oder gefällte Kieselsäure, mineralische, faserförmige Füllstoffe wie Wollastonit oder synthetische, faserförmige Füllstoffe wie Glasfasern, Keramikfasern oder Kunststofffasern eingesetzt.

Bei den nicht verstärkenden Füllstoffen handelt es sich bevorzugt um Cristobalit, Quarz, Diatomeenerde, Zirkoniumsilikat, Kalziumsilikat, Tonminerale, wie Smektite, Zeolithe, Natriumaluminiumsilikate, Metalloxide, wie Aluminium- oder Zinkoxide sowie deren Mischoxide, Bariumsulfat, Kalziumkarbonat, Glaspulver, Hohlglaskugeln sowie Kunststoffpulver.

Der Füllstoff kann gemäß einer bevorzugten Ausführungsform der Erfindung ein oberflächenmodifizierter Füllstoff, vorzugsweise eine organisch oberflächenmodifizierter Füllstoff sein. Der Füllstoff kann nach seiner Oberflächenmodifikation, beispielsweise einer Silanisierung, auf seiner Oberfläche funktionelle Gruppen, besitzen.

Das erfindungsgemäße Dentalmaterial kann zur Einstellung bestimmter Eigenschaften zusätzlich so genannte Additive bzw. Modifikatoren als Hilfsstoffe enthalten. Die Allgemeinheit nicht einschränkend sind nachfolgend einige Beispiele genannt: anorganische und/oder organische Farbpigmente bzw. Farbstoffe, Stabilisatoren (wie z. B. substituierte und unsubstituierte Hydroxyaromaten, Tinuvine, Terpinene, Phenothiazin, sogenannte HALS - Hindered Amine Light Stabilizers - und/oder Schwermetallfänger wie EDTA), Weichmacher (wie z. B. Polethylenglykole, Polypropylenglykole, ungesättigte Polyester, Phthalate, Adipate, Sebacate, Phosphorsäureester, Phosphonsäureester und/oder Zitronensäureester), ionenabgebende Substanzen, insbesondere solche die Fluoridionen freisetzen (wie z. B. Natriumfluorid, Kaliumfluorid, Yttriumfluorid, Ytterbiumfluorid und/oder quartäre Ammoniumfluoride), bakterizide oder antibiotisch wirksame Substanzen (wie z. B. Chlorhexidin, Pyridiniumsalze, Penicilline, Tetracycline, Chloramphenicol, antibakterielle Makrolide und/oder Polypeptid-Antibiotika) und/oder Lösungsmittel (wie z. B. Wasser, Aceton, Ethanol, i-Propanol, Butanon und/oder Essigsäureethylester).

Das Abformmaterial kann hydrophile Eigenschaften aufweisen, z.B. wenn als Hilfsstoff Tenside zugesetzt werden oder wenn Polyethergruppen enthalten sind.

Das Abformmaterial kann auch Stoffe zur Einstellung des pH-Wertes enthalten. Dazu zählen bevorzugt Essigsäure, Zitronensäure, Acetyltributylcitrat, Ascorbinsäure, saure Füllstoffe, saure Puffersysteme wie Essigsäure/Natriumacetatpuffer oder Zitronensäure/Citrat-Puffer sowie alkalische Füllstoffe wie z.B. Aluminiumhydroxid, alkalische Puffersysteme wie z.B. Carbonat/Hydrogencarbonat-Puffer, oder alkalische oder saure Ionenaustauscherharze.

Erfindungsgemäß ist die Abformmasse mischergängig, d.h. die Komponenten Bund K können z.B. aus einer 2-Komponenten-Einweg-Kartusche über einen statischen Mischer oder ein Spendegerät (z.B. MixStar® oder Pentamix®) mit dynamischem Mischer gemischt und ausgetragen werden. Mischergängige Abformmaterialien im vorgenannten Sinne liegen üblicherweise zu Beginn der Mischzeit nach ISO 4823 im Konsistenzbereich von größer 26 mm, vorzugsweise größer 30 mm. Während und nach dem Mischen der Komponenten B und K geht die Mischung von ihrer dünneren mischergängigen Ausgangskonsistenz in einer ersten Stufe, die vorzugsweise über einen Zeitraum von mindestens 15 Sekunden, besonders bevorzugt 2 Minuten, zu einer zäheren, plastischen Phase über, bevor sie in einer zweiten Stufe zu ihrer endgültigen elastischen Form aushärtet. In der ersten Stufe greift der Katalysator (e) an die chelatisierenden Gruppen der Verbindung (b) an und es findet eine Ligandenaustauschreaktion statt, die eine gewisse Vernetzung (Viskosität erzeugt). Der Katalysator ist beispielsweise ein Alkoxymetallkomplex- R₄Me, vorzugsweise R₄Ti oder R₄Zr, der an den β-Dicarbonsäureestern oder β- Ketoestern angreift. In dem zweiten Schritt erfolgt dann die vollständige Vernetzung durch Additionsreaktion der Organohydrogenpolysiloxane (c) mit der Verbindung mit mindestens zwei Alkenylgruppen (a), vorzugsweise unter Verwendung eines Platin-Katalysators (d). Die Komponenten werden vorzugsweise so ausgewählt, dass die Ligandenaustausch- und die Additionsreaktion bei 10 bis 40°C stattfinden, so dass die Reaktionen insbesondere bei Mund- und Raumtemperatur durchgeführt werden können.

Die Erfindung umfasst die erfindungsgemäße Abformmasse in allen beschriebenen Zuständen, einschließlich nach dem Mischen, insbesondere nach dem Mischen der Komponenten B und K und dem Aushärten. Die Mischung im ausgehärteten Zustand erfüllt vorzugsweise die Anforderungen, die nach ISO 4823 an ein elastomeres Abformmaterial im ausgehärteten Zustand gestellt werden, wie zum Beispiel die Rückstellung nach Verformung.

Erfindungsgemäß sind die Komponenten B und K mehr als 3 Monate, vorzugsweise mehr als 6 Monate, weiter vorzugsweise mehr als 12 Monate, besonders bevorzugt mehr als 24 Monate lagerstabil. Lagerstabil im Sinne vorliegender Erfindung bedeutet, dass unter üblichen Lagerbedingungen (Raumtemperatur, trockene Lagerung), zu keinen Reaktionsverlusten kommt, insbesondere zu keinen Reaktionsverlusten, die mit einer Verschlechterung der mechanischen Eigenschaften des Abformmaterials einhergehen.

Die Erfindung betrifft auch ein Verfahren zur Herstellung von Abformungen von abzuformenden Gegenständen.

Erfindungsgemäß wird in dem Verfahren in einem ersten Schritt die Komponenten (a) - (e) der erfindungsgemäßen Abformmasse gemischt, in einem zweiten Schritt die Mischung mit einer Oberfläche eines abzuformenden Gegenstandes in Kontakt gebracht und anschließend die Abformung abgenommen.

Vorzugsweise werden in dem ersten Schritt die Komponenten B und K aus einem Behälter, beispielsweise einer Kartusche über einen Mischer ausgetragen und gemischt. Während des Mischvorgangs und danach geht die Mischung in einen ersten Zustand über, der über die Verarbeitungszeit (vorzugsweise mindestens 15 Sekunden) andauert, in dem die Viskosität der Abformmasse erhöht ist (heavy bodied bis putty Konsistenz), wonach eine Abformung von dem abzuformenden Gegenstand genommen wird. Anschließend geht die Abformmasse in einen nächsten, festen und elastischen Zustand über, in dem das Abformergebnis festgehalten ist, sodass die Abformung vom Gegenstand entfernt werden kann.

Die Erfindung wird nachfolgend ohne Einschränkung der Allgemeinheit anhand von Ausführungsbeispielen erläutert. Die Werte, soweit nicht näher spezifiziert, wurden nach ISO 4823 bestimmt.

### I. erfindungsgemäße Beispiele

### Beispiel 1: Herstellung von Polydimethylsiloxanen, die Chelatgruppen enthalten

In einem Zweihalskolben werden 500 g Tegomer HSi 2311 (α, ω-Hydroxyalkylpolydimethyl-siloxan, 200 mmol, Goldschmidt) und 66,3 g Acetessigsäure-tert.-butylester (419 mmol, Fluka) für 3 h auf 140 °C unter Rühren erwärmt. Während dieser Zeit wird entstehendes tert.-Butanol abdestilliert. Das Reaktionsgemisch wird abgekühlt und flüchtige Bestandteile im Vakuum bei 0,1 mbar und 55 °C entfernt. Ausbeute: 527,54 g. Acetoessigsäureester modifiziertes Polydimethylsiloxan (FT-IR, H-NMR)

### Beispiel 2: Herstellung von Polydimethylsiloxanen, die Chelatgruppen enthalten

In einem Zweihalskolben mit Tropftrichter und CaCl₂-Trockenrohr werden 23,31 g Tegomer HSi 2311 (9 mmol, Goldschmidt) mit 20 mL destilliertem Toluol verdünnt. Unter Rühren werden 1,52 mL destilliertes Pyridin (18 mmol) zugegeben. Im Tropftrichter werden 2 mL Malonsäuremonomethylesterchlorid (18 mmol, Fluka) mit 5 ml destilliertem Toluol verdünnt. Diese Lösung wird langsam über den Tropftrichter zu der Mischung aus Tegomer HSi 2311, Pyridin und Toluol getropft. Nach beendeter Zugabe wird über Nacht bei RT gerührt. Der entstandene Niederschlag wird abfiltriert. Die organische Phase wird 2 x mit 10 mL gesättigter NaHCO₃ extrahiert. Anschließend wird die organische Phase über Na₂SO₄ getrocknet. Am Rotationsverdampfer wird Toluol entfernt und der Rückstand bei 50 °C im Vakuum bei 0,08 mbar von flüchtigen Bestandteilen befreit. Ausbeute: 17,2 g Malonsäuremonomethylester modifiziertes Polydimethylsiloxan (FT-IR, H-NMR)

### Beispiel 3

Je 1 g Acetessigsäureester modifizierte Polydimethylsiloxan aus Beispiel 1 wird mit unterschiedlichen Mengen an Tetra-n-propylzirkonat (Tyzor NPZ, Du Pont) gemäß nachfolgenden Beispielen 3.1, 3.2, 3.3 und 3.4 versetzt. Die Viskosität der Mischung wird an einem Rheometer bei einer Schubspannung von 500 Pa gemessen (DSR von Rheometrics, Geometrie: parallele Platte, Durchmesser: 25 mm, Spaltbreite: 0,1 mm, Temperatur: 23 °C).

| | Beispiel 3.1 | Beispiel 3.2 | Beispiel 3.3 | Beispiel 3.4 |
|---|---|---|---|---|
| Acetoessigsäureester modifiziertes PDMS | 1 g | 1 g | 1 g | 1 g |
| Tyzor NPZ | - | 0,05 g | 0,1 g | 0,2 g |
| Viskosität [Pa·s] | 0,06 | 0,18 | 0,63 | 15693 |

Mit steigendem Anteil an Tetra-n-propylzirkonat stehen auch mehr Metallatome zur Verfügung, die durch die Chelatgruppen komplexiert werden können. Dadurch wird das Molekulargewicht bzw. die Kettenlänge der Polymere erhöht, was sich in der steigenden Viskosität widerspiegelt.

### Beispiel 4: Herstellung einer Basispaste 1 (B1)

In einem Labormischer werden 17,5 Gew. % eines vinylgestoppten Polydimethylsiloxans (65000 mPas, Vinylgehalt 0,03 mmol/g), 4,3 Gew. % eines vinylgestoppten Polydimethylsiloxans (1650000 mPas, Vinylgehalt 0,02 mmol/g), 2,2 Gew. % eines methylgestoppten Polydimethylsiloxans (1000 mPas), 0,2 Gew. % eines Polyhydrogen-methylsiloxans (230 mPas, SiH-Gehalt 2,3 mmol/g), 0,6 Gew. % eines Polyhydrogenmethylsiloxans (40 mPas, SiH-Gehalt 4,3 mmol/g), 3,9 Gew. % Vaseline, 6,0 Gew. % Paraffin und 10 Gew. % Acetoessigsäureester modifiziertes Polydimethylsiloxan aus dem erfindungsgemäßen Beispiel 1 vorgelegt. In diese Mischung werden 16,5 Gew. % Diatomeenerde, 37,2 Gew. % hydrophobiertes Cristobalitmehl, 1 Gew. % hydrophobierte, pyrogene Kieselsäure (BET-Oberfläche 140 m²/g) und 0,6 Gew. % eines Ultramarinpigments eingearbeitet und die Mischung bis zur Homogenität gerührt.

### Beispiel 5: Herstellung einer Katalysatorpaste 1 (K1)

In einem Labormischer werden 5,7 Gew. % eines vinylgestoppten Polydimethylsiloxans (1000 mPas, Vinylgehalt 0,13 mmol/g), 12,7 Gew. % eines vinylgestoppten Polydimethylsiloxans (65000 mPas, Vinylgehalt 0,03 mmol/g), 5,7 Gew. % eines vinylgestoppten Polydimethylsiloxans (1650000 mPas, Vinylgehalt 0,02 mmol/g), 2,4 Gew. % eines methylgestoppten Polydimethylsiloxans (1000 mPas), 3,8 Gew. % Vaseline, 5,7 Gew. % Paraffin, 15,1 Gew. % Diatomeenerde, 41,9 Gew. % hydrophobiertes Cristobalitmehl, 0,18 Gew. % Titandioxid, 0,02 Gew. % 1,3-Divinyl-1,1,3,3-tetramethyldisiloxan, 0,8 Gew. % eines Platin-Katalysators, gelöst in einem vinylgestoppten Polydimethylsiloxan (1000 mPas, Vinylgehalt 0,13 mmol/g, Pt-Gehalt der Lösung = 2 Gew. %) und 6,0 Gew. % Tetra-n-propylzirkonat (Tyzor NPZ, Du Pont) bis zur Homogenität vermischt.

### Beispiel 6: Herstellung einer Katalysatorpaste 2 (K2)

In einem Labormischer werden 5,7 Gew. % eines vinylgestoppten Polydimethylsiloxans (1000 mPas, Vinylgehalt 0,13 mmol/g), 12,7 Gew. % eines vinylgestoppten Polydimethylsiloxans (65000 mPas, Vinylgehalt 0,03 mmol/g), 5,6 Gew. % eines vinylgestoppten Polydimethylsiloxans (1650000 mPas, Vinylgehalt 0,02 mmol/g), 2,4 Gew. % eines methylgestoppten Polydimethylsiloxans (1000 mPas), 3,8 Gew. % Vaseline, 5,7 Gew. % Paraffin, 15,1 Gew. % Diatomeenerde, 41,8 Gew. % hydrophobiertes Cristobalitmehl, 0,18 Gew. % Titandioxid, 0,02 Gew. % 1,3-Divinyl-1,1,3,3-tetramethyldisiloxan, 0,8 Gew. % eines Platin-Katalysators, gelöst in einem vinylgestoppten Polydimethylsiloxan (1000 mPas, Vinylgehalt 0,13 mmol/g, Pt-Gehalt der Lösung = 2 Gew. %) und 6,2 Gew. % Tetra-n-butyltitanat (Tyzor TnBT, Du Pont) bis zur Homogenität vermischt.

### Beispiel 7

Im Handmix (Anmischen auf einer ebenen Unterlage mittels üblichen Dentalspatels) wurden die folgenden Werte für die Pasten B1, K1, K2 der erfindungsgemäßen Beispiele 4-6 und deren Mischungen ermittelt:
Konsistenz B1: 31 mm
Konsistenz K1: 32 mm
Konsistenz K2: 33 mm
Mischkonsistenz 5 Tl. B1 + 1 Tl. K1: 21 mm
Mischkonsistenz 5 Tl. B1 + 1 Tl. K2: 22,5 mm

Verarbeitungsbreite 5 Tl. B1 + 1 Tl. K1: 3:15 - 3:30 min:sec (inkl. 1 min Mischzeit)
Verarbeitungsbreite 5 Tl. B1 + 1 Tl. K2: 3:00 min:sec (inkl. 1 min Mischzeit)

Erhärtungszeit 5 Tl. B1 + 1 Tl. K1: 4:30 min:sec (inkl. 1 min Mischzeit)
Erhärtungszeit 5 Tl. B1 + 1 Tl. K2: 4:00 - 4:15 min:sec (inkl. 1 min Mischzeit)

Aus der Kartusche wurden die folgenden Werte ermittelt:
- aus Kartusche (5 Tl. B1 + 1 Tl. K2) mit 15 mm/min (Mix-Star^{®} Mischsystem, DMG)Vorschub:
   maximale Temperatur beim Anmischen: 31,5 °C
- aus Kartusche (5 Tl. B1 + 1 Tl. K2) mit 24 mm/min Vorschub (Pentamix^{®}, 3M Espe):
   maximale Temperatur beim Anmischen: 31,5 °C

### Beispiel 8: Herstellung einer Basispaste 2 (B2)

In einem Labormischer werden 24,1 Gew. % eines vinylgestoppten Polydimethylsiloxans (65000 mPas, Vinylgehalt 0,03 mmol/g), 0,7 Gew. % eines Polyhydrogen-methylsiloxans (40 mPas, SiH-Gehalt 4,3 mmol/g), 3,9 Gew. % Vaseline, 6,1 Gew. % Paraffin und 10 Gew. % Acetoessigsäureester modifiziertes Polydimethylsiloxan aus Beispiel 1 vorgelegt. In diese Mischung werden 16,5 Gew. % Diatomeenerde, 38,1 Gew. % hydrophobiertes Cristobalitmehl und 0,6 Gew. % eines Ultramarinpigments eingearbeitet und die Mischung bis zur Homogenität gerührt.

### Beispiel 9: Herstellung einer Katalysatorpaste 3 (K3)

In einem Labormischer werden 8,0 Gew. % eines vinylgestoppten Polydimethylsiloxans (1000 mPas, Vinylgehalt 0,13 mmol/g), 12,7 Gew. % eines vinylgestoppten Polydimethylsiloxans (65000 mPas, Vinylgehalt 0,03 mmol/g), 5,6 Gew. % eines vinylgestoppten Polydimethylsiloxans (1650000 mPas, Vinylgehalt 0,02 mmol/g), 3,8 Gew. % Vaseline, 5,6 Gew. % Paraffin, 15,1 Gew. % Diatomeenerde, 42,2 Gew. % hydrophobiertes Cristobalitmehl, 0,02 Gew. % 1,3-Divinyl-1,1,3,3-tetramethyldisiloxan, 0,78 Gew. % eines Platin-Katalysators, gelöst in einem vinylgestoppten Polydimethylsiloxan (1000 mPas, Vinylgehalt 0,13 mmol/g, Pt-Gehalt der Lösung = 2 Gew. %) und 6,2 Gew. % Tetra-n-butyltitanat (Tyzor TnBT, Du Pont) bis zur Homogenität vermischt.

### Beispiel 10

Im Handmix wurden die folgenden Werte für die Pasten B2 und K3 der erfindungsgemäßen Beispiele 8 und 10 und deren Mischung ermittelt:
Konsistenz B2: 29,5 mm
Konsistenz K3: 34 mm

Verarbeitungsbreite 5 Tl. B2 + 1 Tl. K3: 4:45 min:sec (inkl. 1 min Mischzeit)
Erhärtungszeit 5 Tl. B2 + 1 Tl. K3: 4:45 - 5:00 min:sec (inkl. 1 min Mischzeit)
Shore A nach 24 h 5 Tl. B2 + 1 Tl. K3: 36

Aus der Kartusche wurden die folgenden Werte ermittelt:
aus Kartusche (5 Tl. B2 + 1 Tl. K3) mit 15 mm/min Vorschub:
Verarbeitungsbreite: 3:30 min:sec (inkl. 1 min Mischzeit)
Erhärtungszeit: 5:00 min:sec (inkl. 1 min Mischzeit)
bleibende Verformung; 1,3 %
Verformung unter Druck: 6,3 %
Mischkonsistenz: 24 mm
maximale Temperatur beim Anmischen: 31,0 °C

Wie aus den ermittelten Werten der Beispiele 7 und 10 ersichtlich, sind die erfindungsgemäßen Basis- und Katalysatorpasten für ein händisches mischen sowie für die Verwendung in üblichen Kartuschen geeignet und weisen Verarbeitungsparameter auf, wie sie insbesondere bei Dentalanwendungen erwünscht sind.

### II. Vergleichsbeispiele (nicht erfindungsgemäß)

Basis- und Katalysatorpaste für eine nicht erfindungsgemäße additionsvernetzende Abformmassen werden wie folgt hergestellt:

### Beispiel 11: Herstellung einer Basispaste 1 (B1)

In einem Labormischer werden 19,0 Gew. % eines vinylgestoppten Polydimethylsiloxans (1000 mPas, Vinylgehalt 0,13 mmol/g), 21,7 Gew. % eines vinylgestoppten Polydimethylsiloxans (10000 mPas, Vinylgehalt 0,05 mmol/g), 10,9 Gew. % eines methylgestoppten Polydimethylsiloxans (10 mPas), 6,0 Gew. % eines Polyhydrogenmethylsiloxans (230 mPas, SiH-Gehalt 2,3 mmol/g) mit 0,2 Gew. % eines Ultramarinpigments, 4,3 Gew. % einer hydrophobierten, pyrogenen Kieselsäure (BET-Oberfläche 140 m²/g) und 37,9 Gew .% eines hydrophobierten Cristobalitmehls bis zur Homogenität vermischt.

### Beispiel 12: Herstellung einer Katalysatorpaste 1 (K1)

In einem Labormischer werden 22,4 Gew. % eines vinylgestoppten Polydimethylsiloxans (1000 mPas, Vinylgehalt 0,13 mmol/g), 22,4 Gew. % eines vinylgestoppten Polydimethylsiloxans (10000 mPas, Vinylgehalt 0,05 mmol/g), 11,2 Gew. % eines methylgestoppten Polydimethylsiloxans (10mPas), 0,3 Gew. % eines Platin-Katalysators, gelöst in einem vinylgestoppten Polydimethylsiloxan (1000 mPas, Vinylgehalt 0,13 mmol/g, Pt-Gehalt der Lösung = 2 Gew. %) mit 4,5 Gew. % einer hydrophoben, pyrogenen Kieselsäure (BET-Oberfläche 140 m²/g) und 39,2 Gew. % eines hydrophoben Cristobalitmehls bis zur Homogenität vermischt.

### Beispiel 13: Herstellung einer Basispaste 2 (B2)

In einem Labormischer wurden 92,5 Gew. % der Basispaste 1 aus Beispiel 11 mit 7,5 Gew.% des Aminogruppen enthaltenden Polydimethylsiloxans TEGO IS 4111 (Goldschmidt) bis zur Homogenität vermischt.

### Beispiel 14: Herstellung einer Katalysatorpaste 2 (K2)

In einem Labormischer wurden 92,5 Gew. % der Katalysatorpaste 1 aus Beispiel 12 mit 7,5 Gew. % des Aminogruppen enthaltenden Polydimethylsiloxans TEGO IS 4111 bis zur Homogenität vermischt.

### Beispiel 15: Kreuzexperimente mit den Pasten B1, K1, B2 und K2 der Beispiele 11-14

In einem Kreuzexperiment wurden die Basis- und Katalysatorpasten B1, K1, B2, und K2 der vorgenannten Vergleichsbeispiele 11-14 im Gewichtsverhältnis 1:1 vermischt und die Verarbeitungsbreite und die Erhärtungszeit nach Herstellung sowie nach Lagerung bei 23 °C bestimmt (s. nachfolgende Tabelle). Mit diesen Versuchen soll der Einfluss der AminoFunktion auf additionsvernetzende Silikone untersucht werden.

| Kombination | nach Herstellung | | nach Lagerung ^{a)} | |
|---|---|---|---|---|
| | Verarbeitungsbreite ^{b)} | Erhärtungszeit ^{c)} | Verarbeitungsbreite ^{b)} | Erhärtungszeit ^{c)} |
| B1+K1 (Kontrolle) | 3:15 min:sec | 4:30 min:sec | 2:45 - 3:00 min:sec | 4:00 min:sec |
| B1+K2 | nicht bestimmt | ca. 180 min | ca. 120 min | < 16 h |
| B2+K1 | nicht bestimmbar | keine Aushärtung | nicht bestimmbar | keine Aushärtung |
| B2+K2 | nicht bestimmbar | keine Aushärtung | nicht bestimmbar | keine Aushärtung |

| | | | | |
|---|---|---|---|---|
| a) Lagerdauer 83 Tage b) inkl. 1:00 min:sec Mischzeit, gemessen bei 23 °C c) inkl. 1:00 min:sec Mischzeit, gemessen bei 32 °C | | | | |

Aus den Versuchen des Kreuzexperimentes können folgende Schlüsse gezogen werden:

Enthält die Katalysatorpaste eine Verbindung mit Aminogruppen (K2), so findet in Bezug auf die aminogruppenfreie Kontrolle (K1+B1) eine deutlich verlangsamte Abbindung statt (B1+K2) bzw. findet überhaupt nicht statt (B2+K2). Als Ursache wird eine eingangs erläuterte Wechselwirkung der Aminogruppen mit dem Pt-Atom des Platin-Katalysators angenommen, so dass die Hydrosilylierung der Vinylgruppen nur noch sehr langsam abläuft. Enthält die Basispaste eine Verbindung mit Aminogruppen (B2), härten die angemischten Pasten nicht mehr aus (B2+K1 und B2+K2).

### Beispiel 16: Herstellung einer Basispaste 3 (B3)

Die Basispaste 3 vorliegenden Vergleichsbeispiels 16 entspricht in der Herstellung und Zusammensetzung der Basispaste 1 des erfindungsgemäßen Beispiels 4 mit dem Unterschied, dass die 10 Gew.% des Acetoessigsäureester modifizierten Polydimethylsiloxans durch das Aminogruppen modifizierte Polydimethylsiloxan Tego IS 4111 ersetzt wurden.

### Beispiel 17: Herstellung einer Katalysatorpaste 3 (K3)

Die Katalysatorpaste 3 vorliegenden Vergleichsbeispiels 17 entspricht in der Herstellung und Zusammensetzung der Katalysatorpaste 1 des erfindungsgemäßen Beispiels 5.

### Beispiel 18

Im Handmix wurden die folgenden Werte für die Pasten B3 und K3 der Vergleichsbeispiele Beispiele 16 und 17 und deren Mischung ermittelt:
Konsistenz B3: 29 mm
Konsistenz K3: 36 mm

Mischkonsistenz 5 Tl. B3 + 1 Tl. K3: 20 mm
Verarbeitungsbreite 5 Tl. B3 + 1 Tl. K3: 3: nicht feststellbar (inkl. 1 min Mischzeit)
Erhärtungszeit 5 Tl. B3 + 1 Tl. K3: > 40 min (inkl. 1 min Mischzeit)

Im Unterschied zu den Mischungen der erfindungsgemäßen Beispiele 7 und 10 konnte im vorliegenden Vergleichsbeispiel nach der Anmischung beider Pasten B3 und K3 kein verwendbarer, zweistufiger Aushärtemechanismus nachgewiesen werden. Die Verarbeitungsbreite ist nicht nachweisbar und die Erhärtungszeit ist zu langsam.

## Patentansprüche

1. Abformmasse mit
a) mindestens einer Verbindung mit mindestens zwei Alkenylgruppen als Komponente (a),
b) mindestens einer Verbindung mit mindestens einer chelatisierenden Gruppe als Komponente (b),
c) mindestens einem Organohydrogenpolysiloxan als Komponente (c),
d) mindestens einem Hydrosilylierungskatalysator als Komponente (d) und
e) mindestens eine Verbindung mit chelatisierbarem Metallatom als Komponente (e),
wobei die chelatisierende Gruppe der Komponente (b) keine reaktiven Gruppen aufweist, die mit der Komponente (c) und/oder der Komponente (d) reagieren können und die chelatisierende Gruppe der Verbindung der Komponente (b) eine Dicarbonylgruppe ist.

2. Abformmasse nach Anspruch 1, **dadurch gekennzeichnet, dass** die Dicarbonylgruppe eine 1,3-Dicarbonylgruppe wie ein β-Dicarbonsäureester oder β- Ketoester ist.

3. Abformmasse nach Anspruch 1 oder 2, **dadurch gekennzeichnet, dass** die Verbindungen der Komponente b) Verbindungen der Formeln
R⁵ₐR¹_{b}SiO- (SiR¹₂O) _{c}- (SiR¹R⁵O) _{d}-SiR¹_{b}-R⁶ₐ
und
R⁵ aR¹_{b}SiO- [ (SiR¹₂O) _{c}- (SiR¹R⁵O) _{d}- (R⁷)_{g}]ₕ-OSiR¹_{b}R⁵ₐ
mit
R¹= lineares oder verzweigtes Alkyl, Fluoralkyl, Cycloalkyl oder Aryl;
R²= lineares oder verzweigtes Alkylen, Fluoralkylen, Cycloalkylen oder Arylen;
R³= lineares oder verzweigtes Alkylen mit 1 bis 10 C-Atomen, Cycloalkylen oder Arylen;
R⁴= lineares oder verzweigtes Alkyl, Cycloalkyl, Aryl, NR¹₂, NHR¹ oder Alkoxy;
R⁵= R⁴-CO-R³_{f}-CO-Xₑ-R²-;
R⁶= R⁴-CO-R³_{f}-CO-Xₑ-R²;
R⁷= SiR¹₂-R²-Xₑ-CO-R³_{f}-CO-Xₑ-R²-SiR¹₂;
X= O oder NR¹;
a= 0 bis 3; b= 3-a; c= 0 bis 10.000; d= 0 bis 500; e= 0 oder 1; f= 0 oder 1; g= 1 bis 100 und h= 1 bis 1000 sind.

4. Abformmasse nach Anspruch 3, **dadurch gekennzeichnet, dass** R¹ eine Methylgruppe ist.

5. Abformmasse nach Anspruch 3 oder 4, **dadurch gekennzeichnet, dass** R³ eine Methylengruppe ist.

6. Abformmasse nach einem der vorangehenden Ansprüche, **dadurch gekennzeichnet, dass** die chelatisierende Gruppe der Komponente (b) keine reaktiven Gruppen ausgewählt aus der Gruppe bestehend aus Hydroxylalkylgruppe, Aminogruppe und Carboxylgruppe aufweist.

7. Abformmasse nach einem der vorangehenden Ansprüche, **dadurch gekennzeichnet, dass** die Abformmasse aus mindestens zwei Komponenten B und K besteht, wobei die Komponente B die Komponenten (a), (b) und (c) und die Komponente K die Komponenten (d) und (e) enthält.

8. Abformmasse nach Anspruch 7, **dadurch gekennzeichnet, dass** die Komponenten B und K mehr als 3 Monate lagerstabil sind.

9. Abformmassen erhältlich durch Mischen der Komponenten B und K gemäß Anspruch 7.

10. Abformmasse nach Anspruch 9, **dadurch gekennzeichnet, dass** während des Mischens und/oder nach dem Mischen der Komponenten B und K die Mischung in einer ersten Stufe von einer dünneren mischergängigen Ausgangskonsistenz zu einer zäheren, plastischen Phase übergeht, bevor es in einer zweiten Stufe zu seiner endgültigen elastischen Form aushärtet.

11. Gehärtete Abformmasse, erhältlich aus einer Abformmasse nach einem der vorangehenden Ansprüche.

12. Verfahren zur Herstellung einer Abformung von einem abzuformenden Gegenstand, **dadurch gekennzeichnet, dass** eine Abformmasse nach einem der Ansprüche 1 bis 10 verwendet wird, wobei in einem ersten Schritt die Komponenten (a) - (e) gemischt werden, in einem zweiten Schritt die Mischung mit einer Oberfläche eines abzuformenden Gegenstandes in Kontakt gebracht und anschließend die Abformung abgenommen wird.

13. Verwendung einer Verbindung mit mindestens einer chelatisierenden Gruppe die ein β-Dicarbonsäureester oder β-Ketoester ist, als Komponente einer Abformmasse gemäss Anspruch 1 oder 2.

14. Verwendung nach Anspruch 13, **dadurch gekennzeichnet, dass** die Verbindung eine Verbindung der Formeln
R⁵ₐR¹_{b}SiO- (SiR¹₂O) _{c}- (SiR¹R⁵O) _{d}-SiR¹_{b}-R⁶ₐ
und
R⁵ₐR¹_{b}SiO-[(SiR¹₂O)_{c}-(SiR¹R⁵O)_{d}-(R⁷)_{g}]ₕ-OSiR¹_{b}R⁷ₐ
mit
R¹= lineares oder verzweigtes Alkyl, Fluoralkyl, Cycloalkyl oder Aryl;
R² = lineares oder verzweigtes Alkylen, Fluoralkylen, Cycloalkylen oder Arylen;
R³ = Methylen;
R⁴ = lineares oder verzweigtes Alkyl, Cycloalkyl, Aryl, NR¹₂, NHR¹ oder Alkoxy;
R⁵= R⁹-CO-R³_{f}-CO-Xₑ-R²-;
R⁶= R⁴-CO-R³_{f}-CO-Xₑ-R²:
R⁷= SiR¹₂-R²-Xₑ-CO-R³_{f} -CO-Xₑ-R²- SiR¹₂;
X= O oder NR¹;
a= 0 bis 3; b= 3-a; c= 0 bis 10.000; d= 0 bis 500; e= 0 oder 1; f= 1; g= 1 bis 100 und h= 1 bis 1000 ist.

15. Verwendung nach Anspruch 14, **dadurch gekennzeichnet, dass** R¹ eine Methylgruppe ist.

16. Verwendung nach Anspruch 14 oder 15, **dadurch gekennzeichnet, dass** R³ eine Methylengruppe ist.

## Claims

1. An impression composition comprising
a) at least one compound with at least two alkenyl groups as component (a),
b) at least one compound with at least one chelating group as component (b),
c) at least one organohydrogenpolysiloxane as component (c),
d) at least one hydrosilylation catalyst as component (d) and
e) at least one compound with a chelatable metal atom as component (e),
wherein the chelating group of component (b) comprises no reactive groups which are capable of reacting with component (c) and/or component (d) and the chelating group of the component (b) compound is a dicarbonyl group.

2. An impression composition according to claim 1, **characterised in that** the dicarbonyl group is a 1,3-dicarbonyl group such as a β-dicarboxylic acid ester or β-keto ester.

3. An impression composition according to claim 1 or claim 2, **characterised in that** the component b) compounds are compounds of the formulae
R⁵ₐR¹_{b}SiO-(SiR¹₂O)_{c}-(SiR¹R⁵O)_{d}-SiR¹_{b}-R⁶ₐ
and
R⁵ₐR¹_{b}SiO-[(SiR¹₂O)_{c}-(SiR¹R⁵O)_{d}-(R⁷)_{g}]ₕ-OSiR¹_{b}R⁵ₐ
with
R¹= linear or branched alkyl, fluoroalkyl, cycloalkyl or aryl;
R² = linear or branched alkylene, fluoroalkylene, cycloalkylene or arylene;
R³ = linear or branched alkylene with 1 to 10 C atoms, cycloalkylene or arylene;
R⁴ = linear or branched alkyl, cycloalkyl, aryl, NR¹₂, NHR¹ or alkoxy;
R⁵ = R⁴ -CO-R³ _{f}-CO-Xₑ-R² -;
R⁶ = R⁴-CO-R³_{f}-CO-Xₑ-R²;
R⁷ = SiR¹₂-R²-Xₑ-CO-R³_{f}-CO-Xₑ-R²-SiR¹₂;
X = O or NR¹;
a = 0 to 3; b = 3-a; c = 0 to 10,000; d = 0 to 500;
e = 0 or 1; f = 0 or 1; g = 1 to 100 and h = 1 to 1000.

4. An impression composition according to claim 3, **characterised in that** R¹ is a methyl group.

5. An impression composition according to claim 3 or claim 4, **characterised in that** R³ is a methylene group.

6. An impression composition according to any one of the preceding claims, **characterised in that** the chelating group of component (b) comprises no reactive groups selected from the group consisting of hydroxyalkyl group, amino group and carboxyl group.

7. An impression composition according to any one of the preceding claims, **characterised in that** the impression composition consists of at least two components B and K, wherein component B contains components (a), (b) and (c) and component K contains components (d) and (e).

8. An impression composition according to claim 7, **characterised in that** components B and K are stable in storage for more than 3 months.

9. An impression composition obtainable by mixing components B and K according to claim 7.

10. An impression composition according to claim 9, **characterised in that**, during and/or after mixing of components B and K, the mixture changes in a first stage from a thinner, mixer-processable initial consistency into a more viscous, plastic phase, before it cures in a second stage into its definitive elastic form.

11. A cured impression composition obtainable from a impression composition according to any one of the preceding claims.

12. A method for producing an impression of an article of which an impression is to be made, **characterised in that** an impression composition according to any one of claims 1 to 10 is used, wherein in a first step components (a)-(e) are mixed, in a second step the mixture is brought into contact with a surface of an article of which an impression is to be made and then the impression is removed.

13. Use of a compound comprising at least one chelating group which is a β-dicarboxylic acid ester or β-keto ester as a component of an impression composition according to claim 1 or claim 2.

14. Use according to claim 13, **characterised in that** the compound is a compound of the formulae
R⁵ₐR¹_{b}SiO- (SiR¹₂O)_{c}-(SiR¹R⁵O)_{d}-SiR¹_{b}-R⁶ₐ
and
R⁵ₐR¹_{b}SiO- [(SiR¹₂O)_{c}-(SiR¹R⁵O)_{d}-(R⁷)_{g}]ₕ-OSiR¹_{b}R⁵ₐ
with
R¹ = linear or branched alkyl, fluoroalkyl, cycloalkyl or aryl;
R² = linear or branched alkylene, fluoroalkylene, cycloalkylene or arylene;
R³ = linear or branched alkylene with 1 to 10 C atoms, cycloalkylene or arylene;
R⁴ = linear or branched alkyl, cycloalkyl, aryl, NR¹₂, NHR¹ or alkoxy;
R⁵ = R⁴-CO-R³ _{f}-CO-Xₑ-R²-;
R⁶ = R⁴-CO-R³_{f}-CO-Xₑ-R²;
R⁷ = SiR¹₂-R²-Xₑ-CO-R³_{f}-CO-Xₑ-R²-SiR¹₂;
X = O or NR¹;
a = 0 to 3; b = 3-a; c = 0 to 10,000; d = 0 to 500;
e = 0 or 1; f = 0 or 1; g = 1 to 100 and h = 1 to 1000.

15. Use according to claim 14, **characterised in that** R¹ is a methyl group.

16. Use according to claim 14 or claim 15, **characterised in that** R³ is a methylene group.

## Revendications

1. Matière pour empreinte avec
a) au moins un composé avec au moins deux groupes alkylène comme composant (a),
b) au moins un composé avec au moins un groupe de chélation comme composant (b),
c) au moins un organohydrogénopolysiloxane comme composant (c),
(d) au moins un catalyseur d'hydrosilylation comme composant (d), et
(e) au moins un composé avec un atome métallique pouvant être chélaté comme composant (e),
où le groupe de chélation du composant (b) ne comporte aucun groupe réactif, qui pourrait réagir avec le composant (c) et/ou le composant (d) et le groupe de chélation du composant (b) est un groupe dicarbonyle.

2. Matière pour empreinte selon la revendication 1, **caractérisée en ce que** le groupe dicarbonyle est un groupe 1,3-dicarbonyle, comme un ester d'acide β-dicarbonique ou un β-cétoester.

3. Matière pour empreinte selon la revendication 1 ou 2, **caractérisée en ce que** les composés du composant (b) sont des composés des formules :
R⁵ₐR¹_{b}SiO-(SiR¹₂O)_{c}-(SiR¹R⁵O)_{d}-SiR¹_{b}-R⁶ₐ
et
R⁵ₐR¹_{b}SiO-[(SiR¹₂O)_{c}-(SiR¹R⁵O)_{d}-(R⁷)_{g}]ₕ-OSiR¹_{b}-R⁵ₐ
avec
R¹ = alkyle linéaire ou ramifié, fluoroalkyle, cycloalkyle ou aryle ;
R² = alkylène linéaire ou ramifié, fluoroalkylène, cycloalkylène ou amylène
R³ = alkylène linéaire ou ramifié ayant 1 à 10 atomes C, cycloalkylène ou arylène ;
R⁴ - alkyle linéaire ou ramifié, cycloalkyle, aryle, NR¹₂, NHR¹ ou alcoxy ;
R⁵ = R⁴-CO-R³_{f}-CO-Xₑ-R²- ;
R⁶ = R⁴-CO-R³_{f}-CO-Xₑ-R²-;
R⁷ = SiR¹₂-R²-Xₑ-CO-R³_{f}-CO-Xₑ-R²-SiR¹₂;
X = O ou NR¹ ;
a = 0 à 3 ; b = 3-a ; c = 0 à 10 000 ; d = 0 à 500 ; e = 0 ou 1 ; f = 0 ou 1 ; g = 1 à 100 et h = 1 à 1000.

4. Matière pour empreinte selon la revendication 3, **caractérisée en ce que** R¹ est un groupe méthyle.

5. Matière pour empreinte selon la revendication 3 ou 4, **caractérisée en ce que** R³ est un groupe méthylène.

6. Matière pour empreinte selon l'une des revendications précédentes, **caractérisée en ce que** le groupe de chélation du composant (b) ne présente aucun groupe réactif choisi parmi le groupe consistant en un groupe hydroxyalkyle, un groupe amino et un groupe carboxyle.

7. Matière pour empreinte selon l'une des revendications précédentes, **caractérisée en ce que** la matière pour empreinte consiste en au moins deux composants B et K, où le composant B contient les composants (a), (b) et (c) et le composant K contient les composants (d) et (e).

8. Matière pour empreinte selon la revendication 7, **caractérisée en ce que** les composants B et K sont stables pendant plus de 3 mois.

9. Matière pour empreinte pouvant être obtenue par mélange des composants B et K selon la revendication 7.

10. Matière pour empreinte selon la revendication 9, **caractérisée en ce que** pendant le mélange et/ou après le mélange des composants B et K, le mélange passe, dans un premier temps, d'une consistance de départ fluide, mélangeable à une phase plastique, plus visqueuse, avant de durcir en sa forme élastique finale dans un deuxième temps.

11. Matière pour empreinte durcie, obtenue à partir d'une matière pour empreinte selon l'une des revendications précédentes.

12. Procédé de réalisation d'une empreinte d'un objet à mouler, **caractérisé en ce qu'**une matière pour empreinte selon l'une des revendications 1 à 10 est utilisée, où dans une première étape, les composants (a)-(e) sont mélangés, dans une deuxième étape, le mélange est mis en contact avec une surface d'un objet à mouler et ensuite, l'empreinte est enlevée.

13. Utilisation d'un composé avec au moins un groupe de chélation, qui est un ester d'acide β-dicarbonique ou un β-cétoester, comme composant d'une matière pour empreinte selon la revendication 1 ou 2.

14. Utilisation selon la revendication 13, **caractérisée en ce que** le composé est un composé des formules :
R⁵ₐR¹_{b}SiO-(SiR¹₂O)_{c}-(SiR¹R⁵O)_{d}-SiR¹_{b}-R⁶ₐ
et
R⁵ₐR¹_{b}SiO-[(SiR¹₂O)_{c}-(SiR¹R⁵O) _{d}-(R⁷)g]ₕ-OSiR¹_{b}-R⁵ₐ
avec
R¹ = alkyle linéaire ou ramifié, fluoroalkyle, cycloalkyle ou aryle ;
R² = alkylène linéaire ou ramifié, fluoroalkylène, cycloalkylène ou arylène ;
R³ - méthylène ;
R⁴ = alkyle linéaire ou ramifié, cycloalkyle, aryle, NR¹₂, NHR¹ ou alcoxy ;
R⁵ = R⁴-CO-R³ _{f}-CO-Xₑ-R²- ;
R⁶ = R⁴-CO-R³_{f}-CO-Xₑ-R²-;
R⁷ = SiR¹₂-R²-Xₑ-CO-R³_{f}-CO-Xₑ-R²-SiR¹₂ ;
X = O ou NR¹ ;
a = 0 à 3; b = 3-a ; c = 0 à 10 000 ; d = 0 à 500 ; e = 0 ou 1 ; f = 1 ; g = 1 à 100 et h = 1 à 1000.

15. Utilisation selon la revendication 14, **caractérisée en ce que** R¹ est un groupe méthyle.

16. Utilisation selon la revendication 14 ou 15, **caractérisée en ce que** R³ est un groupe méthylène.
